(19) Europäisches Patentamt / European Patent Office / Office européen des brevets

(11) **EP 3 022 670 B1**

(12) **EUROPEAN PATENT SPECIFICATION**

(45) Date of publication and mention of the grant of the patent:
**12.08.2020 Bulletin 2020/33**

(51) Int Cl.:
**G16H 50/70** *(2018.01)*      **G16H 50/20** *(2018.01)*

(21) Application number: **14747414.2**

(22) Date of filing: **26.06.2014**

(86) International application number:
**PCT/IB2014/062611**

(87) International publication number:
**WO 2015/008178 (22.01.2015 Gazette 2015/03)**

(54) **IMAGING BASED RESPONSE CLASSIFICATION OF A TISSUE OF INTEREST TO A THERAPY TREATMENT**

BILDGEBUNGSBASIERTE ANTWORTKLASSIFIZIERUNG EINES GEWEBES VON INTERESSE AUF EINE THERAPEUTISCHE BEHANDLUNG

CLASSIFICATION DE LA RÉPONSE D'UN TISSU D'INTÉRÊT À UN TRAITEMENT THÉRAPEUTIQUE BASÉE SUR L'IMAGERIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.07.2013 US 201361846242 P**

(43) Date of publication of application:
**25.05.2016 Bulletin 2016/21**

(73) Proprietor: **Koninklijke Philips N.V.
5656 AG Eindhoven (NL)**

(72) Inventor: **CARMI, Raz
NL-5656 AE Eindhoven (NL)**

(74) Representative: **Philips Intellectual Property & Standards
High Tech Campus 5
5656 AE Eindhoven (NL)**

(56) References cited:

• **G. R. OXNARD ET AL: "Variability of Lung Tumor Measurements on Repeat Computed Tomography Scans Taken Within 15 Minutes", JOURNAL OF CLINICAL ONCOLOGY, vol. 29, no. 23, 5 July 2011 (2011-07-05), pages 3114-3119, XP055157391, ISSN: 0732-183X, DOI: 10.1200/JCO.2010.33.7071**

• **M. NISHINO ET AL: "Developing a Common Language for Tumor Response to Immunotherapy: Immune-Related Response Criteria Using Unidimensional Measurements", CLINICAL CANCER RESEARCH, vol. 19, no. 14, 6 June 2013 (2013-06-06), pages 3936-3943, XP055157316, ISSN: 1078-0432, DOI: 10.1158/1078-0432.CCR-13-0895**

• **JEREMY J. ERASMUS ET AL: "Interobserver and Intraobserver Variability in Measurement of Non-Small-Cell Carcinoma Lung Lesions: Implications for Assessment of Tumor Response", JOURNAL OF CLINICAL ONCOLOGY, vol. 21, no. 13, 1 July 2003 (2003-07-01), pages 2574-2582, XP009181624, DOI: 10.1200/JCO.2003.01.144**

• **M. YANAGAWA ET AL: "Evaluation of Response to Neoadjuvant Chemotherapy for Esophageal Cancer: PET Response Criteria in Solid Tumors Versus Response Evaluation Criteria in Solid Tumors", THE JOURNAL OF NUCLEAR MEDICINE, vol. 53, no. 6, 11 May 2012 (2012-05-11), pages 872-880, XP055157552, ISSN: 0161-5505, DOI: 10.2967/jnumed.111.098699**

• **ADRIEN DEPEURSINGE ET AL: "Comparative Performance Analysis of State-of-the-Art Classification Algorithms Applied to Lung Tissue Categorization", JOURNAL OF DIGITAL IMAGING ; THE JOURNAL OF THE SOCIETY FOR COMPUTER APPLICATIONS IN RADIOLOGY, SPRINGER-VERLAG, NE, vol. 23, no. 1, 4 November 2008 (2008-11-04), pages 18-30, XP019764523, ISSN: 1618-727X**

**(Cont. next page)**

- **LEI BI ET AL: "Automated and Robust PERCIST-based Thresholding framework for whole body PET-CT studies", 34TH ANNUAL INTERNATIONAL CONFERENCE OF THE IEEE EMBS, 28 August 2012 (2012-08-28), - 1 September 2012 (2012-09-01), pages 5335-5338, XP032464142, ISSN: 1557-170X, DOI: 10.1109/EMBC.2012.6347199**

**Description**

The following generally relates to imaging and more particularly to imaging based response classification of a tissue of interest for a therapy treatment such as a tumor therapy treatment, Alzheimer disease therapy treatment, and/or other therapy treatment.

Monitoring a response of a tissue of interest of a subject to a therapy treatment can facilitate effective treatment of the tissue of interest. For example, distinguishing, for one or more subjects, a tissue of interest that responds to a particular treatment from a tissue of interest not responding to the particular treatment can facilitate maximizing an effectiveness of treatment of the tissue of interest. For instance, for a subject with a tissue of interest that is responding to a particular treatment, the treatment can continue, and that is not responding to the particular treatment, the treatment can be changed or discontinued.

An imaging based assessment of the therapy treatment can provide an indication of the effectiveness of the treatment and has been used in therapy management. Anatomical imaging modalities such as computed tomography (CT) and magnetic resonance imaging (MRI) allow for an accurate assessment of changes in tumor size over the course of a therapy. The World Health Organization (WHO) criteria and the Response Evaluation Criteria in Solid Tumors (RECIST) have provided methodologies for assessing treatment response.

These methodologies have led to the adoption of anatomical imaging modalities in determining the efficacy of treatment for solid tumors in drug development and for regulatory approval, where reproducible quantitative results are of particular importance. Morphological criteria for complete response (CR), partial response (PR), stable disease (SD) and progressive disease (PD) have been established for the RECIST guidelines. Similar analysis schemes are frequently applied in tumor-tracking procedures of individual subjects.

With the increasing adoption of metabolic imaging with positron emission tomography (PET) or PET / CT, particularly with Fluorodeoxyglucose ($^{18}$F) or fludeoxyglucose ($^{18}$F) (also referred to as $^{18}$F-FDG or FDG) imaging in initial staging and follow-up of a variety of cancers, improvements for the RECIST criteria have been suggested in order to base them not only on tumor size, as measured by morphological imaging modalities like CT, but also on tumor metabolism parameters, like standardized uptake value (SUV), as measured by PET.

For PET, therapy response assessment criteria includes PET Response Criteria In Solid Tumors (PERCIST) and the International Harmonization Project for Response Criteria in Lymphoma, which have been proposed based on PET metabolic criteria. Since many newer cancer therapies may have different effect on tumor morphology during treatment, good tumor response may be associated predominantly with a decrease in metabolism or in other functional parameters such as blood perfusion, even in the absence of a major reduction in tumor size.

RECIST guidelines in practice evaluate response by assessing a set of measurable target lesions, and, an optional additional group of non-target or new lesions. This is performed in baseline and in follow-up imaging studies and the relations or changes between the follow-up results of different times are evaluated. Unfortunately, applying RECIST consistently is challenging, e.g., due to inter-observer variability among physicians such as oncologists and radiologists, and due to the inherent uncertainties in the selection and measurement of target lesions, which has been achieved manually or using automated software.

Particularly, a tumor lesion may have very irregular and complicated shape and/or metabolic distribution, which may introduce inherent uncertainty in extracting lesion characteristics and parameters. Improvements in automatic lesion segmentation and analysis algorithms and corresponding software have been developed and include improved reporting, tracking and comparison schemes. Other sources of such uncertainties and inaccuracies include the choice of tracked lesion selection by a user, the imaging modality and protocol, contrast agent injection, image quality, noise, patient position, the evaluation tools, etc.

Imaging indications of therapy response in non-cancerous diseases include, for example, the amount and distribution in the brain of beta amyloid plaque associated with dementia and Alzheimer disease, which can be imaged with PET and MRI.

However, even the state of the art tracking clinical application tools do not thoroughly evaluate the uncertainties and inaccuracies of the overall diagnosis. This includes current computerized methods which enable more automatic and reliable tumor-tracking workflow. Unfortunately, the uncertainties and inaccuracies can lead to erroneous diagnosis and therapy course selection.

Aspects described herein address the above-referenced problems and others.

The following describes an approach for assessing a response of a tissue of interest to a therapy treatment. In one instance, the assessment is based on parameter measurement uncertainties and/or sets of probabilities of response classes. The assessment includes combining the sets of probabilities of response classes, and determining an overall response class for the therapy treatment based on the combination.

In one aspect, a method for determining a final response class of tissue of interest to a therapy treatment includes obtaining a first set of probabilities of response classes and at least a second set of probabilities of the response classes. The method further includes combining the first and the at least second sets of probabilities of the response classes, thereby generating a combined set of probabilities of the response classes. The method further includes determining the final response class of the tissue of interest to the therapy treatment from a plurality of predefined response classes based on the combined set of probabilities of the response classes. The method further includes generating a signal indicative of the final response class.

In another aspect, a therapy response classifier includes a combined response classes probability determiner that combines a first set and at least a second set of probabilities of response classes for tissue of interest to a therapy treatment, generating a combined set of probabilities of the response classes and a response class determiner that determines a final response class of the tissue of interest to the therapy treatment from the response classes based on the combined set of probabilities of the response classes.

In another aspect, a computer readable storage medium is encoded with computer readable instructions, which, when executed by a processer, causes the processor to: obtain at least two sets of probabilities of response classes, wherein the response classes include at least two classes indicative of two different response of tissue of interest to a therapy treatment, combine the at least two sets of probabilities, and determine a final response class of the tissue of interest to the therapy treatment based on the combined at least two sets of probabilities.

The invention may take form in various components and arrangements of components, and in various steps and arrangements of steps. The drawings are only for purposes of illustrating the preferred embodiments and are not to be construed as limiting the invention.

FIGURE 1 schematically illustrates an example system 102 including at least a therapy response classifier 104 and an imaging system(s) 106.

FIGURE 2 schematically illustrates an example of the therapy response classifier of FIGURE 1.

FIGURE 3 illustrates examples of different measurements for a single parameter of tissue of interest at one point in time and a corresponding uncertainty.

FIGURE 4 illustrates an example of visualizing the different measurements for tissue of interest over multiple points in time and uncertainties at each point in time.

FIGURE 5 illustrates an approach for determining a set of probabilities of response classes for a parameter measurement.

FIGURE 6 illustrates an approach for combining sets of probabilities of response classes.

FIGURE 7 illustrates an example method in accordance with the embodiments herein.

The following describes an approach for determining, based on imaging data, a response class of a tissue of interest to a therapy treatment.

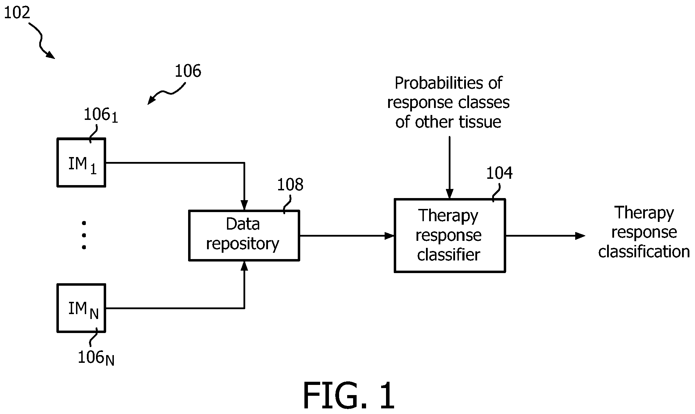

FIGURE 1 illustrates a system 102 including a therapy response classifier 104 in connection with N imaging systems 106 (where N is a positive number equal to or greater than one), including an imaging system $106_1$, ..., $106_N$ and a data repository 108, which can be a separate storage component (as shown), part of the imaging systems 106. The N imaging systems 106 includes one or more of a computed tomography (CT) scanner, a magnetic resonance imaging (MRI) scanner, a positron emission tomography (PET) scanner, a hybrid or multi-modality (e.g., a PET/CT) scanner, and/or other imaging system(s).

One or more different imaging techniques can be performed on the same scanner and/or different scanner. For example, anatomical CT and functional dynamic contrast-enhanced CT can be performed with the same CT scanner. The N imaging systems can also include different imaging instances of the same technique.

The data repository 108 can be used to store imaging data generated by the N imaging systems 106. The data repository 108 can include storage local to one or more of the N imaging systems 106 and/or storage remote from the one or more of the N imaging systems 106. The data repository 108 can include a radiology information system (RIS), a hospital information system (HIS), a picture archiving and communication system (PACS), an electronic medical record (EMR), a server, a database, and/or other data storage device.

The imaging data stored in the data repository 108 at least includes imaging data acquired at two different points in time during a therapy treatment (e.g., radiation therapy, chemotherapy, disease-targeted medication, etc.). For instance, the imaging data may include data acquired pre and post a therapy treatment, after each fraction of a therapy treatment, at two different points in time after a therapy treatment, etc. Such data can be from a same or different imaging system of the imaging systems 106.

The therapy response classifier 104 classifies a response of the tissue of interest to the therapy treatment. As described in greater detail below, this includes obtaining an uncertainty for each of a plurality of parameter measurements, evaluating the parameters and their uncertainties based on predetermined classification criteria, determining probabilities for each different possible class, combining the probabilities for different imaging systems, groups of tissue of interest, different response point, etc. based on predetermined combining criteria, and determining a final classification of the response based on the combined probabilities.

Optionally, the final classification is also based on probabilities of response classes for other tissue (i.e., tissue other than the tissue of interest) and/or already determined probabilities of response classes for the tissue of interest. Examples of suitable classifications include, but are not limited to the Response Evaluation Criteria in Solid Tumors (RECIST) and the PET Response Criteria In Solid Tumors (PERCIST). RECIST response classes include, for example, complete response (CR), partial response (PR), stable disease (SD) and progressive disease (PD). Other response classes are also contemplated herein. For example, other classifications include absent or present, new tissue of interest, etc.

Using the uncertainties and the probabilities as described herein may reduce response class inaccuracies relative to a configuration in which the therapy response classifier 104 does not consider the uncertainties and the probabilities. As such, the described approach is well suited for monitoring a response of a tumor, Alzheimer disease, etc. to therapy to determine an effectiveness of the therapy and to validate and/or adjust the treatment. For example, where the therapy response classification of the therapy response classifier 104 indicates that the tissue of interest is responding as desired to a therapy treatment, the therapy treatment can be continued. However, where the therapy response classification of the therapy response classifier 104 indicates that the tissue of interest is not responding as desired to the therapy treatment, the therapy treatment can be changed to a different therapy treatment or discontinued.

It is to be appreciated that the therapy response classifier 104 can be implemented via one or more computer processors (e.g., a central processing unit (CPU), a microprocessor, etc.) executing one or more computer executable instructions embedded or encoded on computer readable storage medium such as physical memory and excluding non-transitory medium. At least one of the computer executable instructions can alternatively be carried by a carrier wave, signal, and other transitory medium.

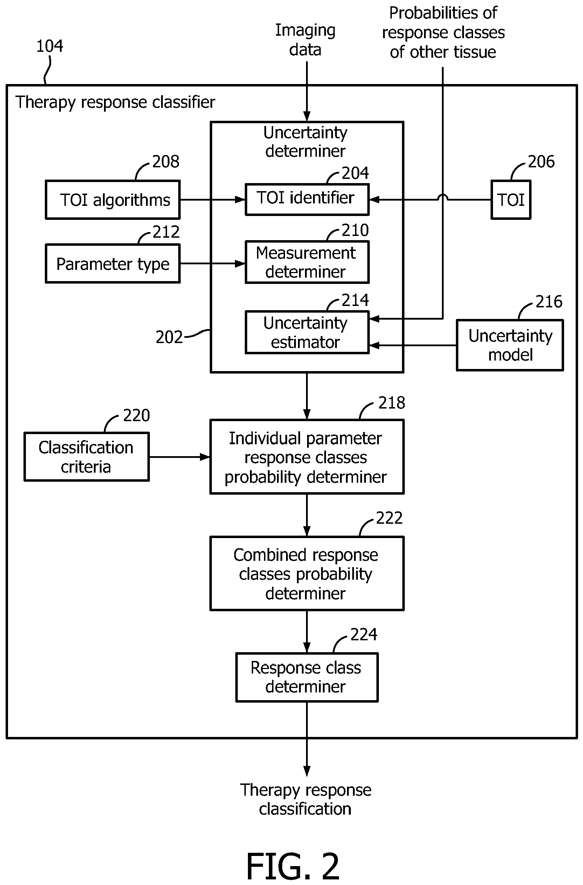

FIGURE 2 illustrates an example of the therapy response classifier 104. As discussed herein and shown in FIGURE 2, the therapy response classifier 104, in one instance, receives, as an input, imaging data (e.g., from the data repository 108, one or more of the imaging system 106, and/or other device) and, optional probabilities of response of classes of other tissue (or tissue other than the tissue of interest).

An uncertainty determiner 202 determines an uncertainty for each of the parameter measurements based on the input imaging data.

The uncertainty determiner 202 includes a tissue of interest (TOI) identifier 204 that identifies one or more tissues of interest in the imaging data based on a tissue of interest (TOI) 206 and tissue of interest (TOI) algorithms 208. The TOI 206 can be, for example, one or more tumors being treated with the therapy treatment, where each tumor is identified using two or more of the TOI algorithms 208. The TOI identifier 204 can identify the tissue(s) of interest based on an automated segmentation algorithm and/or in connection with user interaction, e.g., based on input indicative of a user identified contour in the imaging data.

The uncertainty determiner 202 further includes a parameter measurement determiner 210 that determines one or more parameter measurements of the identified tissue of interest based on one or more predetermined parameter types 212. This includes multiple measurements for the same parameter, where each measurement is based on different identified tissue of interest for the same TOI 206, which is identified using a different algorithm of the TOI algorithms 208. Examples of parameter types include anatomical size (e.g. volume, longest length, shortest length), mean, maximum and/or other intensity value (e.g. SUV in PET, HU in CT, contrast-agent enhancement), etc.

The uncertainty determiner 202 further includes an uncertainty estimator 214 that estimates an uncertainty for each of the parameters based on the measurements of each of the parameters and an uncertainty model 216, which can be a Gaussian and/or other distribution. For example, if the parameter type is a length of the tissue of interest, the uncertainty can be a probability distribution width, and the uncertainty model 216 can be a Gaussian distribution function.

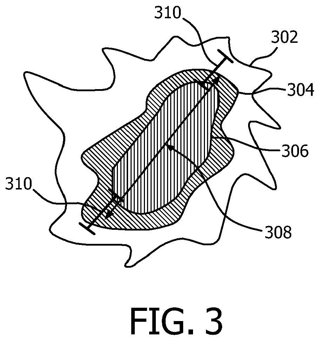

For instance, FIGURE 3 shows an example in which three different algorithms of the TOI algorithms 208 are used to identify three different boundaries 302, 304, and 306 of a tissue of interest. For example, the three different algorithms may be associated with three different image intensity thresholds. The uncertainty estimator 214 estimates a length 308 with an uncertainty 310, which is in terms of a width of a Gaussian probability distribution, in this example. The uncertainty estimation can be done, for example, by automatic algorithm which minimizes a cost function.

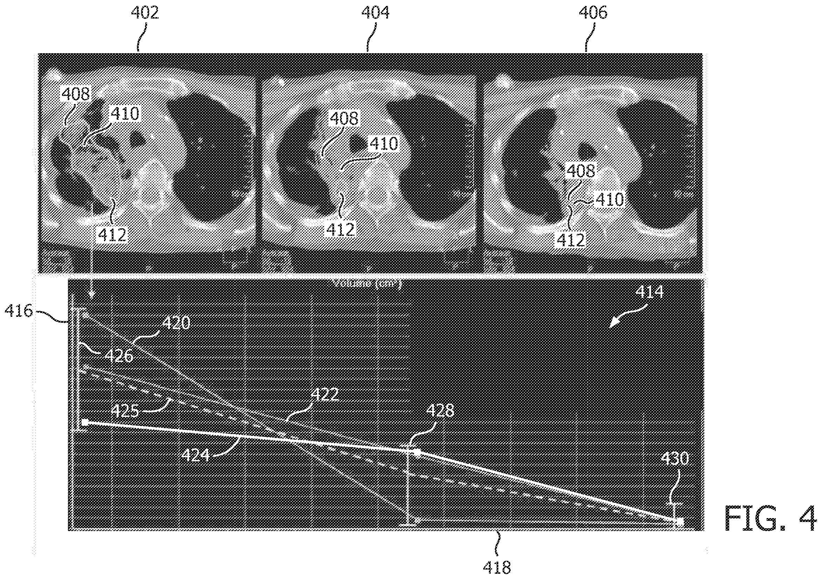

The uncertainty determiner 202 outputs an uncertainty for each of the parameter measurements. FIGURE 4 shows a non-limiting example for visualizing the uncertainties. Images 402, 404 and 406 are acquired at three different moments in time. Each of the images 402, 404 and 406 includes three contours 408, 410 and 412, all for the same tissue of interest, but determined using different TOI algorithms of the TOI algorithms 208.

A graph 414 includes curves for the contours 408, 410 and 412. In the graph 414, a y-axis 416 represents tissue of interest volume and an x-axis 418 represents time. A first curve 420 represents a change in a volume of the tissue of interest over time based on a first algorithm, a second curve 422 represents a change in the volume of the tissue of interest over the same time based on a second algorithm, and a third curve 424 represents a change in the volume of the tissue of interest over the same time based on a third algorithm.

A fourth curve 425 represents the three curves 420, 422 and 424. It is to be appreciated that different techniques can be used to generate the fourth curve 425 and the resulting curve from each technique may differ from a curve from another technique. The graph 414 also includes uncertainties 426, 428, and 430 for each of the three different images 402, 404 and 406.

Returning to FIGURE 2, an individual parameter response classes probability determiner 218 evaluates the uncertainties of the parameter measurements based on predetermined classification criteria 220. Such evaluation can be based on image modality, group of tissue of interest, and/or other grouping. The individual parameter response classes probability determiner 218 outputs a set of probabilities of response classes (e.g., CR, PR, SD and PD, according to pre-defined criteria such as RECIST) for each parameter measurement, or for each group of parameter measurement, according to the pre-defined criteria.

The following describes a non-limiting example of determining probabilities of response classes for each group of parameter measurement.

In this example, $x_i$ represents parameter measurements of each combination of time, tissue of interest and parameter type where $i$ is a running index, $dx_i$ represents all corresponding estimated uncertainties, $mdx_i$ represents all corresponding uncertainty model types, and $v_1 = v(t_1) = $ a set (i.e. the group) of all the $x_i$ parameter values for all the $i$'s parameters that are evaluated in time point $t_1$. $v_0$ is a set of reference or baseline parameters.

Furthermore, a function F(v) represents a function that acts on a set of specific values of parameters (e.g. sum of tissue of interest lengths, or a combination of lengths and mean image intensity). In one instance, all the same tissue of interest are evaluated in time points $t_1$ and $t_0$. In another instance, all the same tissue of interest are not evaluated in time points $t_1$ and $t_0$; some tissue of interest may not be evaluated in some time points.

A function G ($F_1(v_1)$, $F_0(v_0)$) represents a response criterion function. The function G can be a linear or non-linear (or non-continuous) function, or a set of rules (e.g. RECIST rules). In one instance, the function definition of $F_1$ is identical to that of $F_0$. In another instance, the function definition of $F_1$ is not identical to that of $F_0$. The response criterion defines a response class: s = G ($F_1(v_1)$, $F_0(v_0)$) where s is a response class. Optionally, G can be a function of several time points, i.e. s = $G(F_1(v_1), F_2(v_2), ... F_0(v_0))$

With the uncertainties determined by the uncertainty determiner 202, the individual parameter response classes probability determiner 218 determines a propagation scheme. Where F is a simple sum, e.g., = $x_1 + x_2 + x_3 + ... $ , then

$$dX \text{(in terms of normal probability distribution)} = (dx_1^2 + dx_2^2 + dx_3^2 + \cdots)^{1/2} \text{ In such a case, } dX_1, dX_0, ...$$

define the probability distributions of $F_1(v_1)$, $F_0(v_0)$ ..., and uncertainty propagation can be applied for the function G to calculate the resultant probability distribution of the classes s.

If the uncertainty $dX$ or all the $dx_i$ represent standard deviation of normal distribution probability (or any other defined distribution), then the probabilities of s can be calculated by a Monte-Carlo simulation process, a direct numerical calculation (and where G can be any function), and/or other approach. For a general calculation description, $r_i$ represents a specific selected possible value of $F_i$, depending on the already estimated distribution of the function results. The probability of this value $p_j(r_i)$ depends on the known distribution model of $F_j$ which defines the function of $p_j$.

If the probability of $r_i$ is known (e.g. $p_j(r_i) = n \cdot exp((r_i^2 - X_j^2)/2/dX_j^2)$ as in a Gaussian model, where $n$ is used for normalization), the individual class probability determiner 210 can select a set (with index $k$) of: $r_1$, $r_0$, ... corresponding to the evaluated parameters (e.g. in a random selection way or as an instance in a serial sequence) and calculate: $P_k(s) = (p_1(r_1) \cdot p_0(r_0) \cdot ...) \cdot G(r_1, r_0, ...)$

This scheme is repeated for several or many different sets $k$ of different $r$'s while choosing the sets according to a proper scheme (effectively spanning uniformly all possible combinations). Since the uncertainties of $F_1(v_1)$ and $F_0(v_0)$ can be calculated once (i.e. $dF_1$ and $dF_0$) (especially if F is a linear function such as a sum), in that case r is a choice in the known distribution of F.

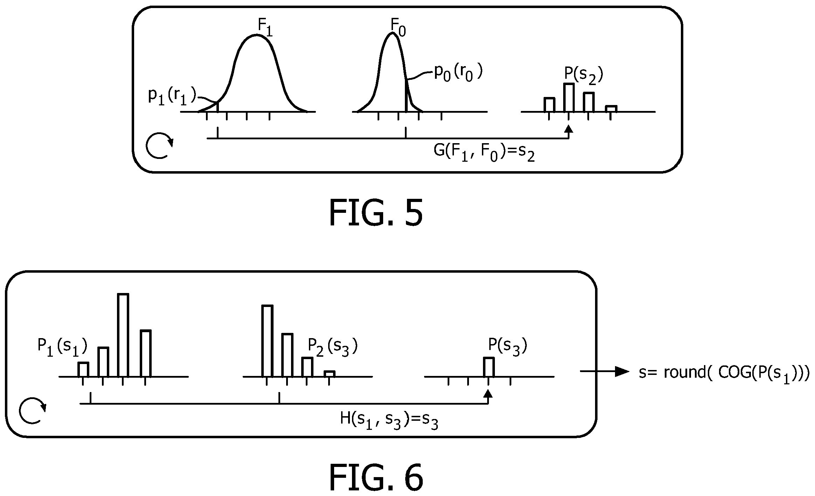

The scheme includes repeating the calculation of $P_k(s)$ (the probability of each defined s) and summing, for each s, all the calculated probabilities (to obtain the final probability of each possible s). This is illustrated in FIGURE 5, where $F_1$ is (for example) the probability distribution of possible total length sum of all tracked lesion in a current evaluation time point, and $F_0$ is (for example) the probability distribution of possible total length sum of all tracked lesion in the reference (baseline) evaluation time point.

In FIGURE 5, $r_1$ and $r_0$ are the selected values within these distributions in one iteration of the calculation. Applying the classification function G with the inputs $r_1$ and $r_0$ gives as the result the class $s_2$. The multiplication of the probabilities $p_1$, and $p_0$ give the probability of $s_2$ in this single iteration. This process is repeated with different values for $r_1$ and $r_0$, which can be randomly and/or otherwise selected. The classification probabilities are normalized at the end of the iterative process.

In a more general case, where F can be a more complex function, $r_i$ represents a specific selected possible parameter value distributed around $x_i$, and that for all $x_i$ in the group v, $R_1$ represents a set of $r_{11}, r_{12}, r_{13}, ..., R_0$ represents a set of $r_{01}, r_{02}, r_{03}, ...,$ and $p(r_i)$ depends on the reported uncertainty model type $mdx_i$ which defines the function of p.

If the probability of $r_i$ is known (e.g. $p(r_i) = n \cdot exp((r_i^2 - x_i^2)/2/dx_i^2)$ in a Gaussian model), a set (with index $k$) of: $r_1$, $r_2$, ... corresponding to the evaluated parameters (e.g. in a random selection way or as an instance in a serial sequence) can be selected and $P_k(s) = (p(r_{11}) \cdot p(r_{12}) \cdot ... \cdot p(r_{01}) \cdot p(r_{02}) \cdot ... ) \cdot G(F_1(R_1), F_0(R_0))$ can be calculated.

This approach is repeated a plurality of times for different sets k of different $r$'s while choosing the sets according to a predetermined scheme (e.g., to effectively span all possible combinations), r can be selected for all the parameters of the sets $v_1$ and $v_0$, ....

Returning to FIGURE 2, a combined response classes probability determiner 222 evaluates the individual probabilities of response classes for all modalities, imaging techniques, independent tissue of interest groups, response time points, etc. The following describes a non-limiting example of an evaluation performed by the combined response classes probability determiner 222.

With the possible response classes $s_1$, $s_2$, $s_3$, ... (for example CR, PR, SD, PD), for a classification 1, the set of probabilities of response classes is [$P_1(s_1)$, $P_1(s_2)$, $P_1(s_3)$, ... ], for a classification 2, the set of probabilities of response classes: [$P_2(s_1)$, $P_2(s_2)$, $P_2(s_3)$, ...], ... $s_a$ represents a selected one of: $s_1$, $s_2$, $s_3$, ... corresponding to sub-classification 1, $s_b$ represents a selected one of: $s_1$, $s_2$, $s_3$, ... corresponding to sub-classification 2, ..., the combined response classes probability determiner 212 can employ a combination criteria standard such as: H = Response criterion ($s_a$, $s_b$, ...), which can be a linear or a non-linear (or non-continuous) function, or a set of rules.

A response class determiner 224 determines a final estimated overall response classification. In one non-limiting instance, the response class determiner 224 determines a final estimated overall response classification by calculating, for a selected specific set of: $s_a$, $s_b$, ..., $P(s) = (P_1(s_a) \cdot P_2(s_b) \cdot ... ) \cdot H(s_a, s_b)$. The response class determiner 224 repeats this for one or more different sets of ($s_a$, $s_b$, ...) . The sets are selected such that a sufficient number of sets is selected according to a predetermined scheme (e.g., effectively spanning all combinations).

The response class determiner 224 repeats the calculation of $P(s)$ (the probability of each defined s and sums, for each s, all the calculated probabilities. As a final classification, the response class determiner 224, in one non-limiting instance, takes the s value with the highest total probability (e.g. the closest to the calculated 'center of gravity' (COG)). This is illustrated in connection with FIGURE 6, which shows response class probability evaluation: $P_1(s_1)$ is the probability of the class $s_1$ from the first modality, and $P_2(s_3)$ is the probability of the class $s_3$ from the second modality.

Applying the classification function H with the inputs $s_1$ and $s_3$ gives as the result the class $s_3$. The multiplication of the probabilities $P_1$ and $P_2$ gives the probability of $s_3$ in this single iteration. The whole process is repeated for all the combinations of choices of s. The final classification probabilities can be normalized at the end of the iterative process. The final class is considered as the one who is closest to the center of gravity of the four probabilities.

The criteria H may be dependent also on external information regarding the patient condition, accuracy of imaging etc. For example, if for a certain disease it is known that PET is more accurate than CT, the PET classification may get higher weight.

In a variation of the above, uncertainties can be used also to select sufficiently appropriate target tissue of interest as the basis for the whole response monitoring evaluation, and to reject lesions with very high parameter uncertainties.

The following describes three example evaluations, which are based on the example RECIST guideline of Table 1.

| Table 1 – Time point response: patients with target (+/– non-target) disease. | | | |
|---|---|---|---|
| Target lesions | Non-target lesions | New lesions | Overall response |
| CR | CR | No | CR |
| CR | Non-CR/non-PD | No | PR |
| CR | Not evaluated | No | PR |
| PR | Non-PD or not all evaluated | No | PR |
| SD | Non-PD or not all evaluated | No | SD |
| Not all evaluated | Non-PD | No | NE |
| PD | Any | Yes or No | PD |
| Any | PD | Yes or No | PD |
| Any | Any | Yes | PD |
| CR = complete response, PR = partial response, SD = stable disease, PD = progressive disease, and NE = inevaluable. | | | |

For a first evaluation, no uncertainties are evaluated. Assuming the target lesions response is PR, and the non-target lesion response is Non-CR/Non-PD. Based on the rules, the overall response is PR. For a second evaluation, uncertainties are evaluated. In this example, the assumed obtained probability distributions are: The target lesion is 0.2\*CR + 0.5\*PR + 0.3\*SD, and the non-target lesion is 0.7\*NonCRNonPD + 0.3\*PD. With this evaluation, the independent mean values of the two groups are still closer to PR and Non-CR/Non-PD which give together the original overall assessment above of PR.

For a third evaluation, the overall response of each combination is multiplied by the joint probabilities of the target and non-target lesion groups, as discussed herein, and the overall response is 0.14\*PR + 0.06\*PD + 0.35\*PR + 0.15\*PD + 0.21\*SD + 0.09\*PD, which equals 0.49\*PR + 0.21\*SD + 0.3\*PD. The center of gravity (COG) of the distributions is (with defining: CR==1, PR==2, SD==3, PD==4): COG= (0.49\*2+0.21\*3+0.3\*4)/(0.49+0.21+0.3), or 2.81, which rounds to SD (round(2.81)=3), so the overall response is SD.

For this example, the final response class of SD is a more accurate reflection of the actual response of the tissue of interest to the therapy treatment than the other two evaluations, which resulted response classes of PR.

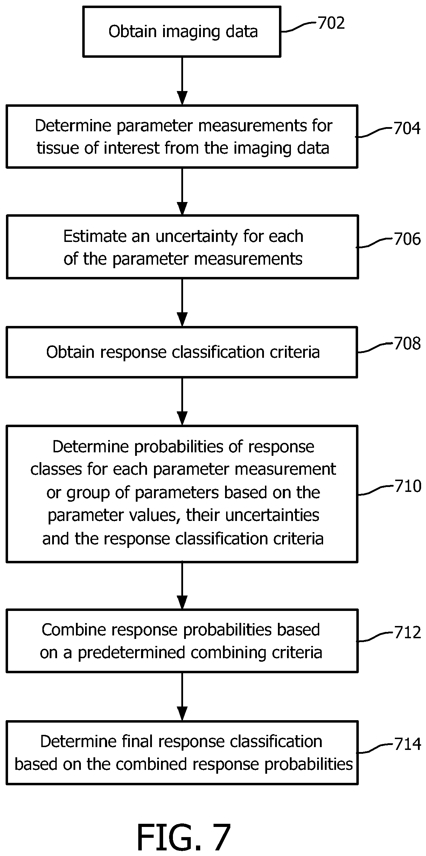

FIGURE 7 illustrates an example method in accordance with the embodiments described herein.

It is to be appreciated that the ordering of the acts in the methods described herein is not limiting. As such, other orderings are contemplated herein. In addition, one or more acts may be omitted and/or one or more additional acts may be included.

At 702, imaging data is obtained.

At 704, parameter measurements are determined for tissue of interest from the imaging data.

At 706, an uncertainty is estimated for each of the parameter measurements.

At 708, response classification criteria is obtained.

At 710, probabilities of response classes are determined for each parameter measurement or group of parameters, based on the parameter values, their uncertainties and the response classification criteria.

At 712, the probabilities of response classes are combined based on predetermined combining criteria.

At 714, a final response class is determined for the tissue of interest based on the combined probabilities of response classes.

The above may be implemented by way of computer readable instructions, encoded or embedded on computer readable storage medium, which, when executed by a computer processor(s), cause the processor(s) to carry out the described acts. Additionally or alternatively, at least one of the computer readable instructions is carried by a signal, carrier wave or other transitory medium.

The invention has been described with reference to the preferred embodiments. Modifications and alterations may occur to others upon reading and understanding the preceding detailed description, insofar as they come within the scope of the appended claims.

**Claims**

1.  A computer implemented method for determining a final response class of tissue of interest to a therapy treatment, comprising:

    determining a parameter measurement for the tissue of interest based on imaging data;
    determining an uncertainty of the parameter measurement;
    obtaining a first set of probabilities of response classes, wherein the response classes include at least a first class indicative of a first response of the tissue of interest to the therapy treatment;
    obtaining at least a second set of probabilities of the response classes, wherein the response classes include at least a second class indicative of a second response of the tissue of interest to the therapy treatment, wherein the first and the second responses are different;
    combining the first and the at least second sets of probabilities of the response classes, thereby generating a combined set of probabilities of the response classes;
    determining at least one of the first or the at least the second set of probabilities based on the uncertainty and predetermined response criteria;
    determining the final response class of the tissue of interest to the therapy treatment from a plurality of predefined response classes based on the combined set of probabilities of the response classes; and
    generating a signal indicative of the final response class.

2.  The method of claim 1, wherein at least one of the first or the at least the second set of probabilities is generated based on imaging data acquired from a same imaging modality.

3.  The method of any of claims 1 to 2, wherein at least one of the first and the at least the second set of probabilities is based on a group of the tissue of interest.

4.  The method of claim 2, wherein at least one of the first and the at least the second set of probabilities is based on a second group of tissue that does not include the tissue of interest.

5.  The method of any of claims 1 to 4, wherein at least one of the first and the at least the second set of probabilities is based on a response time point.

6.  The method of any of claims 1 to 5, wherein determining the final response class includes selecting a response class of the plurality of the response classes with a highest total probability.

7.  The method of any of claims 1 to 6, wherein the predetermined response classification criteria includes response classes from a group consisting of complete response; partial response, stable disease; and progressive disease.

8.  The method of any of claims 1 to 7, wherein determining the final response class comprises:

    randomly selecting a response class of the first set of probabilities;
    randomly selecting a response class of the second set of probabilities;
    combining the probabilities corresponding to the selected response class of the first set of probabilities and the probabilities corresponding to the selected response class of the second set of probabilities based on predetermined combining criteria; and
    repeating, one or more times, the acts of randomly selecting response classes of the first and the second set of probabilities and combining the probabilities.

9.  The method of claim 8, wherein combining the probabilities includes multiplying the probabilities.

10. The method of any of claims 8 to 9, further comprising:
    determining the final response class based on a center of gravity of the probabilities, wherein the final response class is closest to a center of gravity of the probabilities.

11. A therapy response classifier (104) for determining a final response class of tissue of interest to a therapy treatment, comprising:

    a measurement determiner (210) that is configured to determine a parameter measurement of the tissue of interest based on the imaging data;
an uncertainty estimator (214) that is configured to determine an uncertainty of the parameter measurement;
an individual parameter response classes probability determiner (218) that is configured to determine probabilities of response classes for each parameter measurement or group of parameters based on parameter values, parameter value uncertainties, and response classification criteria;
a combined response classes probability determiner (222) that is configured to combine a first set and at least a second set of probabilities of response classes for tissue of interest to a therapy treatment, generating a combined set of probabilities of the response classes, wherein the response classes include at least a first class indicative of a first response of the tissue of interest to the therapy treatment and at least a second class indicative of a second response of the tissue of interest to the therapy treatment, wherein the first and the second responses are different;
a response class determiner (224) that is configured to determine a final response class of the tissue of interest to the therapy treatment from the response classes based on the combined set of probabilities of the response classes.

12. The therapy response classifier of claim 11, wherein the response class determiner is configured to determine a final response class as the response class with a highest total probability.

**Patentansprüche**

1. Eine auf einem Computer auszuführende Methode zum Ermitteln der abschließenden Ansprechklasse des im Rahmen einer therapeutischen Behandlung zu untersuchenden Gewebes, die Folgendes umfasst:

Ermitteln einer Parametermessung für das zu untersuchende Gewebe beruhend auf Bildgebungsdaten;
Ermitteln einer Unsicherheit der Parametermessung;
Abrufen eines ersten Wahrscheinlichkeitssatzes der Ansprechklassen, wobei die Ansprechklassen mindestens eine erste Klasse umfassen, die das erste Ansprechen des zu untersuchenden Gewebes auf die Behandlung angibt;
Abrufen mindestens eines zweiten Wahrscheinlichkeitssatzes der Ansprechklassen, wobei die Ansprechklassen mindestens eine zweite Klasse umfassen, die das zweite Ansprechen des zu untersuchenden Gewebes auf die Behandlung angibt, und wobei sich das erste und zweite Ansprechen unterscheiden;
Kombinieren des ersten und des mindestens zweiten Wahrscheinlichkeitssatzes der Ansprechklassen, um einen kombinierten Wahrscheinlichkeitssatz für die Ansprechklassen zu erhalten;
Ermitteln von mindestens einem der ersten oder mindestens zweiten Wahrscheinlichkeitssätze beruhend auf der Unsicherheit und den vordefinierten Ansprechkriterien;
Ermitteln der abschließenden Ansprechklasse des zu untersuchenden Gewebes unter mehreren vordefinierten Ansprechklassen anhand des kombinierten Wahrscheinlichkeitssatzes für die Ansprechklassen; und
Generieren eines Signals, das auf die abschließende Ansprechklasse hinweist.

2. Die Methode gemäß Anspruch 1, wobei mindestens einer des ersten oder des mindestens zweiten Wahrscheinlichkeitssatzes beruhend auf den Bildgebungsdaten desselben Bildgebungsverfahrens generiert wird.

3. Die Methode gemäß Anspruch 1 und 2, wobei mindestens einer des ersten oder des mindestens zweiten Wahrscheinlichkeitssatzes auf einer Gruppe des zu untersuchenden Gewebes beruht.

4. Die Methode gemäß Anspruch 2, wobei mindestens einer des ersten oder des mindestens zweiten Wahrscheinlichkeitssatzes auf einer zweiten Gewebegruppe beruht, die nicht das untersuchende Gewebe umfasst.

5. Die Methode gemäß einer der Ansprüche 1 bis 4, wobei mindestens einer des ersten oder des mindestens zweiten Wahrscheinlichkeitssatzes auf einem Ansprechzeitpunkt beruht.

6. Die Methode gemäß einer der Ansprüche 1 bis 5, wobei das Ermitteln der abschließenden Ansprechklasse das Auswählen einer Ansprechklasse aus den Ansprechklassen mit der höchsten Gesamtwahrscheinlichkeit umfasst.

7. Die Methode gemäß einer der Ansprüche 1 bis 6, wobei die vordefinierten Ansprechklassifizierungskriterien Ansprechklassen aus einer Gruppe mit vollständigem Ansprechen, partiellem Ansprechen, stabile Erkrankung und fortschreitende Erkrankung umfasst.

8. Die Methode gemäß einer der Ansprüche 1 bis 7, wobei das Ermitteln der abschließenden Ansprechklasse Folgendes umfasst:

zufälliges Auswählen einer Ansprechklasse des ersten Wahrscheinlichkeitssatzes;
zufälliges Auswählen einer Ansprechklasse des zweiten Wahrscheinlichkeitssatzes;
Kombinieren der Wahrscheinlichkeiten für die ausgewählte Ansprechklasse des ersten Wahrscheinlichkeitssatzes und der Wahrscheinlichkeiten für die ausgewählte Ansprechklasse des zweiten Wahrscheinlichkeitssatzes beruhend auf den vordefinierten Kombinationskriterien; und
ein- oder mehrmaliges Wiederholen der zufälligen Auswahl von Ansprechklassen des ersten und des zweiten Wahrscheinlichkeitssatzes und des Kombinierens der Wahrscheinlichkeiten.

9. Die Methode gemäß Anspruch 8, wobei das Kombinierens der Wahrscheinlichkeiten das Multiplizieren der Wahrscheinlichkeiten umfasst.

10. Die Methode gemäß einer der Ansprüche 8 bis 9, wobei diese zudem Folgendes umfasst:
Ermitteln der abschließenden Ansprechklasse beruhend auf dem Schwerpunkt der Wahrscheinlichkeiten, wobei die abschließende Ansprechklasse dem Schwerpunkt der Wahrscheinlichkeiten am nächsten ist.

11. Ein Behandlungs-Ansprechklassierer (104) zum Ermitteln der abschließenden Ansprechklasse des zu untersuchenden Gewebes für eine therapeutische Behandlung, der Folgendes umfasst:

eine Messbestimmung (210), mit der beruhend auf den Bildgebungsdaten eine Parametermessung für das zu untersuchende Gewebe ermittelt wird;
ein Unsicherheitskalkulator (214) zum Ermitteln der Unsicherheit der Parametermessung;
eine Ansprechklassen-Wahrscheinlichkeitsbestimmung für die einzelnen Parameter (218), das die Wahrscheinlichkeiten der Ansprechklassen für einzelne Parametermessungen oder für Gruppen von Parametern beruhend auf den Parameterwerten, den Unsicherheiten der Parameterwerte und den Klassifizierungskriterien ermittelt;
eine Wahrscheinlichkeitsbestimmung für die kombinierten Ansprechklassen (222), die einen ersten und mindestens einen zweiten Wahrscheinlichkeitssatz der Ansprechklassen für das zu untersuchende Gewebe im Rahmen einer Behandlung kombiniert, um einen kombinierten Wahrscheinlichkeitssatz für die Ansprechklassen zu erhalten, wobei die Ansprechklassen mindestens eine erste und eine zweite Klasse umfassen, die jeweils das erste und zweite Ansprechen des zu untersuchenden Gewebes auf die Behandlung angibt, und wobei sich das erste und zweite Ansprechen unterscheiden;
eine Ansprechklassenbestimmung (224), die die abschließende Ansprechklasse des zu untersuchenden Gewebes unter mehreren Ansprechklassen anhand des kombinierten Wahrscheinlichkeitssatzes für die Ansprechklassen ermittelt.

12. Der Behandlungs-Ansprechklassierer gemäß Anspruch 11, wobei die Ansprechklassenbestimmung als abschließende Ansprechklasse die Ansprechklasse mit der höchsten Gesamtwahrscheinlichkeit ermittelt.

**Revendications**

1. Procédé mis en œuvre par ordinateur de détermination d'une classe de réponse finale d'un tissu d'intérêt à un traitement thérapeutique, comprenant :

la détermination d'une mesure de paramètre pour le tissu d'intérêt en fonction des données d'imagerie ;
la détermination d'une incertitude de la mesure de paramètre ;
l'obtention d'un premier ensemble de probabilités de classe de réponse, dans lequel les classes de réponse comprennent au moins une première classe indicative d'une première réponse du tissu d'intérêt au traitement thérapeutique ;
l'obtention d'au moins un second ensemble de probabilités de classe de réponse, dans lequel les classes de réponse comprennent au moins une seconde classe indicative d'une seconde réponse du tissu d'intérêt au traitement thérapeutique, dans lequel les première et seconde réponses sont différentes ;
la combinaison du premier et dudit au moins second ensemble de probabilités de classe de réponse, générant ainsi un ensemble combiné de probabilités de classe de réponse ;
la détermination desdits premier et/ou second ensembles de probabilités en fonction de l'incertitude et des critères de réponse prédéterminés ;

la détermination de la classe de réponse finale du tissu d'intérêt pour le traitement thérapeutique à partir d'une pluralité de classes de réponse prédéfinies en fonction de l'ensemble combiné de probabilités de classe de réponse ; et

la génération d'un signal indicatif de la classe de réponse finale.

2. Procédé selon la revendication 1, dans lequel les au moins premier et/ou second ensembles de probabilités sont générés en fonction des données d'imagerie acquises à partir d'une même modalité d'imagerie.

3. Procédé selon l'une quelconque des revendications 1 à 2, dans lequel les au moins premier et second ensembles de probabilités sont basés sur un groupe de tissus d'intérêt.

4. Procédé selon la revendication 2, dans lequel les au moins premier et second ensembles de probabilités sont basés sur un second groupe de tissus, lequel n'inclut pas le tissu d'intérêt.

5. Procédé selon l'une quelconque des revendications 1 à 4, dans lequel les au moins premier et second ensembles de probabilités sont basés sur un point temporel de réponse.

6. Procédé selon l'une quelconque des revendications 1 à 5, dans lequel la détermination de la classe de réponse finale comprend la sélection d'une classe de réponse de la pluralité de classes de réponse à une probabilité totale supérieure.

7. Procédé selon l'une quelconque des revendications 1 à 6, dans lequel les critères de classification de la réponse prédéterminés comprennent des classes de réponse d'un groupe composé par une réponse complète ;une réponse partielle, une maladie stable ; et une maladie progressive.

8. Procédé selon l'une quelconque des revendications 1 à 7, dans lequel la détermination de la classe de réponse finale comprend :

la sélection aléatoire d'une classe de réponse du premier ensemble de probabilités ;
la sélection aléatoire d'une classe de réponse du second ensemble de probabilités ;
la combinaison des probabilités correspondant à la classe de réponse sélectionnée du premier ensemble de probabilités et des probabilités correspondant à la classe de réponse sélectionnée du second ensemble de probabilités en fonction des critères de combinaison prédéterminés ; et
la répétition, au moins une fois, des étapes de sélection aléatoire des classes de réponse du premier et du second ensemble de probabilités et de combinaison de probabilités.

9. Procédé selon la revendication 8, dans lequel la combinaison des probabilités comprend la multiplication de probabilités.

10. Procédé selon l'une quelconque des revendications 8 à 9, comprenant en outre :
la détermination de la classe de réponse finale en fonction d'un centre de gravité des probabilités, la classe de réponse finale étant la plus proche d'un centre de gravité des probabilités.

11. Classificateur de réponses thérapeutiques (104) permettant de déterminer une classe de réponse finale du tissu d'intérêt pour un traitement thérapeutique, comprenant :

un déterminant de mesure (210), lequel est configuré pour déterminer une mesure de paramètre du tissu d'intérêt en fonction des données d'imagerie ;
un estimateur d'incertitude (214), lequel est configuré pour déterminer une incertitude de la mesure des paramètres ;
un déterminant de probabilité de classe de réponse du paramètre individuel (218), lequel est configuré pour déterminer les probabilités de classe de réponse pour chaque mesure de paramètre ou groupe de paramètres en fonction des valeurs de paramètre, des incertitudes de valeur de paramètre et des critères de classification de réponses ;
un déterminant de probabilité de classe de réponse combinées (222), lequel est configuré pour combiner un premier ensemble et au moins un second ensemble de probabilités de classe de réponse pour le tissu d'intérêt pour un traitement thérapeutique, générant un ensemble combiné de probabilités de classe de réponse, dans lequel les classes de réponse comprennent au moins une première classe indicative d'une première réponse du tissu d'intérêt au traitement thérapeutique et au moins une seconde classe indicative d'une seconde réponse du tissu d'intérêt au traitement thérapeutique, dans lequel les première et seconde réponses sont différentes ; un déterminant de classe de réponse (224), lequel est configuré pour déterminer une classe de réponse finale du tissu d'intérêt pour le traitement thérapeutique à partir des classes de réponse en fonction de l'ensemble combiné de probabilités de classe de réponse.

12. Classificateur de réponses thérapeutiques selon la revendication 11, dans lequel le déterminant de classe de réponse est configuré pour déterminer une classe de réponse finale en tant que classe de réponse à la probabilité totale supérieure.

Probabilities of
response classes
of other tissue

Therapy
response
classification

104

Therapy
response
classifier

108

Data
repository

106

106₁

IM₁

· · ·

IMₙ

106ₙ

102

**FIG. 1**

FIG. 2

FIG. 3

FIG. 4

FIG. 5

FIG. 6

```
┌─────────────────────────┐
│    Obtain imaging data   │──702
└─────────────────────────┘
            │
            ▼
┌─────────────────────────┐
│ Determine parameter      │
│ measurements for         │──704
│ tissue of interest from  │
│ the imaging data         │
└─────────────────────────┘
            │
            ▼
┌─────────────────────────┐
│  Estimate an uncertainty │
│  for each                │──706
│  of the parameter        │
│  measurements            │
└─────────────────────────┘
            │
            ▼
┌─────────────────────────┐
│ Obtain response          │──708
│ classification criteria  │
└─────────────────────────┘
            │
            ▼
┌─────────────────────────┐
│ Determine probabilities  │
│ of response              │
│ classes for each         │
│ parameter measurement    │
│ or group of parameters   │──710
│ based on the             │
│ parameter values, their  │
│ uncertainties            │
│ and the response         │
│ classification criteria  │
└─────────────────────────┘
            │
            ▼
┌─────────────────────────┐
│ Combine response         │
│ probabilities based      │──712
│ on a predetermined       │
│ combining criteria       │
└─────────────────────────┘
            │
            ▼
┌─────────────────────────┐
│ Determine final response │
│ classification           │──714
│ based on the combined    │
│ response probabilities   │
└─────────────────────────┘
```

# FIG. 7